# EUROPÄISCHE PATENTSCHRIFT

(11) **EP 2 300 510 B1**
(45) Veröffentlichungstag und Bekanntmachung des Hinweises auf die Patenterteilung: **07.12.2011**
(21) Anmeldenummer: 09793873.2
(22) Anmeldetag: 26.06.2009
(51) Int. Cl.: C08G 18/10, C08G 18/48, C08G 18/73, C08G 18/75, C08G 18/79, A61L 15/26

(54) **HYDROPHILE, ALIPHATISCHE POLYURETHAN-SCHÄUME**
HYDROPHILIC, ALIPHATIC POLYURETHANE FOAMS
MOUSSES DE POLYURÉTHANE ALIPHATIQUES ET HYDROPHILES

(30) Priorität: 09.07.2008 EP 08012372
(43) Veröffentlichungstag der Anmeldung: 30.03.2011
(73) Patentinhaber: Bayer MaterialScience AG, 51368 Leverkusen (DE)
(72) Erfinder: SCHÖNBERGER, Jan, 42781 Haan (DE); KÖHLER, Burkhard, 34289 Zierenberg (DE); HAAS, Peter, 42781 Haan (DE); NIESTEN, Meike, 51061 Köln (DE)
(86) Internationale Anmeldenummer: PCT/EP2009/004610
(87) Internationale Veröffentlichungsnummer: WO 2010/003559

(56) Entgegenhaltungen:
- EP-A- 1 745 847
- US-A- 2 894 919
- US-A- 4 191 815
- US-A- 5 807 958

## Beschreibung

Die Erfindung betrifft hydrophile, aliphatische Polyurethan-Schäume, die durch Reaktion von speziellen monomerenarmen Präpolymeren und gegebenenfalls oligomeren Isocyanaten in Gegenwart von Wasser und Katalysatoren zugänglich sind. Aufgrund ihrer Absorptionseigenschaften sind die hydrophilen Polyurethan-Schäume insbesondere geeignet zur Herstellung von Wundauflagen, kosmetischen Artikeln oder Inkontinenzprodukten.

Die EP-A 949285 beschreibt die Reaktion von Polyisocyanaten mit primären Diaminen, niedermolekularen Polyolen und hochmolekularen Polyolen. Bei dieser Reaktion ist nicht auszuschließen, dass erhebliche Teile der isocyanatreaktiven Substanzen nicht umgesetzt werden und anschließend aus dem hydrophilen Schaum extrahierbar sind.

Die GB 1571730 beschreibt die Reaktion von Diisocyanaten mit hohem Dampfdruck wie Isophorondiisocyanat (IPDI) und Bis(isocyanatocyclohexyl)methan (HMDI) mit Polyolen. Auch hier bleiben nicht umgesetzte Komponenten zurück. Ferner ist das Arbeiten mit freien, nicht derivatisierten Diisocyanaten aus Sicht der Arbeitshygiene problematisch. In der WO 2004013215 werden ebenfalls leicht flüchtige Diisocyanate umgesetzt.

In der GB 1571730 wie auch in der US 3778390, US 3799898 und FR 2077388 werden als Schaumstabilisatoren siliziumhaltige und siliziumfreie nichtionische, Sulfat-, Phosphat- und Sulfonat-Emulgatoren genannt. Diese weisen jedoch eine geringe Zellverträglichkeit auf. Die Verwendung von Carboxylaten wird nicht erwähnt.

Die WO2003/097727, US 5065752 und US 5064653 beschreiben die Schaumbildungsreaktion von Präpolymeren in Gegenwart von Acrylamid-Acrylsäure-Copolymeren. Diese Produkte sind chemisch nicht angekoppelt und können komplett extrahiert werden, was ebenfalls nicht wünschenswert ist.

In der US 3903232 und US 388941 werden Präpolymere mit Polyethern umgesetzt. Auch hier besteht die Gefahr des Auftretens nicht angekoppelter Polyole. Die US 5296518 beschreibt ebenso die Umsetzung von Präpolymeren mit Polyethern, wobei drei unterschiedliche Polyole eingesetzt werden, was die Wirtschaftlichkeit dieses Verfahrens in Frage stellt. Zudem lässt sich mit dem dort beschriebenen Verfahren nicht sicherstellen, dass keine niedermolekularen Isocyanate im Gemisch verbleiben, was nicht wünschenswert ist. Die Verwendung von Carboxylaten wird nicht erwähnt. Die Herstellung der Präpolymere erfordert zumeist unwirtschaftlich lange Reaktionszeiten. Aufgabe der vorliegenden Erfindung war daher die Bereitstellung eines Verfahrens zur Herstellung hydrophiler, aliphatischer Polyurethan-Schäume, welche insbesondere als Bestandteil einer Wundauflage, eines kosmetischen Artikels oder eines Inkontinenzprodukts eingesetzt werden können, und daher nur wenig extrahierbare Bestandteile aufweisen sollen. Zudem ist es von großer prozesstechnischer Wichtigkeit, dass die Polyurethan-Schäume nach dem Aufschäumen keinen Volumenschrumpf erleiden. Überdies sollen bei deren Herstellung nur Polyisocyanate mit einem niedrigen Dampfdruck, also keine unmodifizierten Diisocyanate, eingesetzt werden. Die hydrophilen, aliphatischen Polyurethan-Schäume sollen überdies eine rasche und hohe Absorption von physiologischer Salzlösung bzw. von Wundflüssigkeit aufweisen, ohne dass hierzu zwingend superabsorbierende Polymere eingesetzt werden müssen. Wundauflagen aus diesen Polyurethan-Schäumen sollen zellverträglich sein (nicht cytotoxisch) und sich in der Anwendung der Wundform optimal anpassen.

Es wurde nun gefunden, dass sich Präpolymere aus aliphatischen Diisocyanaten, vorzugsweise HDI, und Polyethern mit einem Ethylenoxid-Gehalt von mindestens 50 mol% bezogen auf den Gesamtgehalt an Oxyalkyleneinheiten in Mischungen mit Uretdiongruppen- und Isocyanuratgruppen-haltigen Oligomeren auf Basis von Hexamethylendüsocyanat (HDI) mit Wasser in Gegenwart ausgesuchter Aktivatoren und gegebenenfalls Schaumstabilisatoren verschäumen lassen.

Gegenstand der vorliegenden Erfindung ist daher ein Verfahren zur Herstellung hydrophiler, aliphatischer Polyurethan-Schäume, bei dem Zusammensetzungen umfassend
A) isocyanatfunktionelle Präpolymere mit einem Gewichtsanteil an niedermolekularen, aliphatischen Diisocyanaten mit einer Molmasse von 140 bis 278 g/mol von unter 1,0 Gew.- % bezogen auf das Präpolymer, erhältlich durch Umsetzung von
   A1) niedermolekularen, aliphatischen Diisocyanaten einer Molmasse von 140 bis 278 g/mol mit
   A2) di- bis hexafunktionellen, bevorzugt tri- bis hexafunktionellen Polyalkylenoxiden einer OH-Zahl von 22,5 bis 112, bevorzugt von 31,5 bis 56, und einem Ethylenoxidanteil von 50 bis 100 mol%, bevorzugt 60 bis 85 mol%, bezogen auf die Gesamtmenge der enthaltenen Oxyalkylengruppen,
B) gegebenenfalls heterocyclische, 4-Ring- oder 6-Ring-Oligomere von niedermolekularen, aliphatischen Diisocyanaten mit einer Molmasse von 140 bis 278 g/mol,
C) Wasser,
D) gegebenenfalls Katalysatoren,
E) C₈- bis C₂₂-Monocarbonsäuren oder deren Ammonium- oder Alkalisalze oder C₁₂- bis C₄₄-Dicarbonsäuren oder deren Ammonium- oder Alkalisalze,
F) gegebenenfalls Tenside und
G) gegebenenfalls ein- oder mehrwertige Alkohole
bereitgestellt, aufgeschäumt und ausgehärtet werden.

Bevorzugt weisen die in A) eingesetzten Präpolymere einen Restmonomergehalt von unter 0,5 Gew.-% bezogen auf das Präpolymer auf. Dieser Gehalt kann durch entsprechend gewählte Einsatzmengen von A1) und A2) erreicht werden. Bevorzugt ist jedoch der Einsatz des Isocyanats A1) im Überschuss und anschließender, bevorzugt destillativer, Abtrennung nicht umgesetzter Monomere.

Die Herstellung der isocyanatfunktionellen Präpolymere der Komponente A) erfolgt typischerweise durch Umsetzung von einem Val der Polyolkomponente A2) mit einem bis 20 Mol, bevorzugt einem bis 10 Mol, besonders bevorzugt 5 bis 10 Mol, des niedermolekularen, aliphatischen Diisocyanats A1).

Die Umsetzung kann in Gegenwart von Urethanisierungskatalysatoren wie Zinnverbindungen, Zinkverbindungen, Aminen, Guanidinen oder Amidinen, oder in Gegenwart von Allophanatisierungskatalysatoren wie Zinkverbindungen erfolgen.

Die Umsetzung erfolgt typischerweise bei 25 bis 140°C, bevorzugt 60 bis 100°C.

Wurde mit überschüssigem Isocyanat gearbeitet so erfolgt anschließend die Entfernung des Überschusses an niedermolekularem, aliphatischen Diisocyanat bevorzugt durch Dünnschichtdestillation.

Vor, während und nach der Reaktion oder destillativen Abtrennung des Diisocyanatüberschusses können saure oder alkylierende Stabilisatoren, wie Benzoylchlorid, Isophthaloylchlorid, Methyltosylat, Chlorpropionsäure, HCl oder Antioxidantien, wie Di-tert-butylkresol oder Tocopherol zugesetzt werden.

Der NCO-Gehalt der isocyanatfunktionellen Präpolymere A) beträgt bevorzugt 1,5 bis 4,5 Gew.- %, besonders bevorzugt 1,5 bis 3,5 Gew.-% und ganz besonders bevorzugt 1,5 bis 3,0 Gew.-%. Beispiele für niedermolekulare, aliphatischen Diisocyanate der Komponente A1) sind Hexamethylendiisocyanat (HDI), Isophorondiisocyanat (IPDI), Butylendiisocyanat (BDI), Bisisocyanatocyclohexylmethan (HMDI), 2,2,4-Trimethylhexamethylendiisocyanat, Bisisocyanatomethylcyclohexan, Bisisocyanatomethyltricyclodecan, Xylendiisocyanat, Tetramethylxylylendiisocyanat, Norbornandiisocyanat, Cyclohexandiisocyanat oder Diisocyanatododecan, wobei Hexamethylendiisocyanat (HDI), Isophorondiisocyanat (IPDI), Butylendiisocyanat (BDI) und Bis(isocyanatocyclohexyl)methan (HMDI) bevorzugt sind. Besonders bevorzugt sind BDI, HDI, IPDI, ganz besonders bevorzugt Hexamethylendiisocyanat und Isophorondiisocyanat.

Polyalkylenoxide der Komponente A2) sind bevorzugt Copolymere aus Ethylenoxid und Propylenoxid mit einem Ethylenoxidgehalt, bezogen auf die Gesamtmenge der enthaltenen Oxyalkylengruppen, von 50 bis 100 mol%, bevorzugt 60 bis 85 mol%, gestartet auf Polyolen oder Aminen. Geeignete Starter dieser Art sind Glycerin, Trimethylolpropan (TMP), Sorbit, Pentaerythrit, Triethanolamin, Ammoniak oder Ethylendiamin.

Die Polyalkylenoxide der Komponente A2) besitzen typischerweise zahlenmittlere Molekulargewichte von 1000 bis 15000 g/mol, bevorzugt von 3000 bis 8500 g/mol.

Ferner besitzen die Polyalkylenoxide der Komponente A2) OH-Funktionalitäten von 2 bis 6, bevorzugt von 3 bis 6, besonders bevorzugt von 3 bis 4.

Gegebenenfalls einzusetzende Verbindungen der Komponente B) sind heterocyclische, 4-Ring- oder 6-Ring-Oligomere von niedermolekularen, aliphatischen Diisocyanaten mit einer Molmasse von 140 bis 278 g/mol wie Isocyanurate, Iminooxadiazindione oder Uretdione der vorgenannten niedermolekularen, aliphatischen Diisocyanate. Bevorzugt sind heterocyclische 4-Ring-Oligomere wie Uretdione.

Der durch Einsatz der Komponente B) erhöhte Gehalt an Isocyanatgruppen sorgt für ein besseres Aufschäumen durch mehr gebildetes CO₂ aus der Isocyanat-Wasser-Reaktion.

Das als Komponente C) einzusetzende Wasser kann als solches, als Kristallwasser eines Salzes, als Lösung in einem dipolar-aprotischen Lösungsmittel oder auch als Emulsion eingesetzt werden. Bevorzugt wird das Wasser als solches oder in einem dipolar-aprotischen Lösungsmittel eingesetzt. Ganz besonders bevorzugt wird das Wasser als solches eingesetzt.

Zur Beschleunigung der Urethanbildung können in Komponente D) Katalysatoren eingesetzt werden. Dabei handelt es sich typischerweise um die dem Fachmann aus der Polyurethantechnologie bekannten Verbindungen. Bevorzugt sind hier Verbindungen der Gruppe bestehend aus katalytisch aktiven Metallsalzen, Aminen, Amidinen und Guanidinen. Beispielhaft zu nennen sind Zinndibutyldilaurat (DBTL), Zinnoctoat (SO), Zinnacetat, Zinkoctoat (ZO), 1,8-Diazabicyclo[5.4.0]undecen-7 (DBU), 1,5-Diazabicyclo[4.3.0]nonen-5 (DBN), 1,4-Diazabicyclo[3.3.0]octen-4 (DBO), N-Ethylmorpholin (NEM), Triethylendiamin (DABCO), Pentamethylguanidin (PMG), Tetrametylguanidin (TMG), Cyclotetramethylguanidin (TMGC), n-Decyltetramethylguanidin (TMGD), n-Dodecyltetramethylguanidin (TMGDO), Dimethylaminoethyltetramethylguanidin (TMGN), 1,1,4,4,5,5-Hexamethylisobiguanidin (HMIB), Phenyltetramethylguanidin (TMGP) und Hexamethylenoctamethylbiguanidin (HOBG).

Besonders bevorzugt ist der Einsatz von Aminen, Amidinen, Guanidinen oder deren Mischungen als Katalysatoren der Komponente D). Ganz besonders bevorzugt wird 1,8-Diazabicyclo[5.4.0]undecen-7 (DBU) eingesetzt.

In einer bevorzugten Ausführungsform der Erfindung werden in Komponente D) Verbindungen der vorgenannten Art als Katalysatoren eingesetzt.

Als Komponente E) werden Ammonium- und Alkalisalze von C₈- bis C₂₂-Monocarboxylaten oder deren freien Carbonsäuren oder C₁₂- bis C₄₄-Dicarboxylaten oder deren freien Dicarbonsäuren, bevorzugt Kalium- oder Natriumsalze von C₈- bis C₂₂-Monocarboxylaten oder C₁₂- bis C₄₄-Dicarboxylaten und besonders bevorzugt Natriumsalze von C₈- bis C₂₂-Monocarboxylaten eingesetzt.

Beispielsweise geeignete Verbindungen der Komponente E) sind die Ammonium-, Na-, Li- oder K-Salze von Ethylhexansäure, Octansäure, Decansäure, Dodecansäure, Palmitinsäure, Stearinsäure, den Octadecensäuren, den Octadecadiensäuren, den Octadecatriensäuren, Isostearinsäure, Erucasäure, Abietinsäure und ihren Hydrierungsprodukten. Beispiele für C₁₂- bis C₄₄-Dicarbonsäuren bzw. die daraus abgeleiteten Ammonium- und Alkalisalze sind Dodecandisäure, Dodecenyl-, Tetradecenyl-, Hexadecenyl- und Octadecenyl-Bernsteinsäure, C₃₆- und C₄₄-Dimerfettsäuren und ihre Hydrierungsprodukte sowie die entsprechenden Ammonium-, Na-, Li- oder K-Salze dieser Dicarbonsäuren.

Zur Verbesserung der Schaumbildung, Schaumstabilität oder der Eigenschaften des resultierenden Polyurethan-Schaums können Verbindungen der Komponente F) eingesetzt werden, wobei solche Additive grundsätzlich alle an sich bekannten anionischen, kationischen, amphoteren und nichtionischen Tenside sowie Mischungen hieraus sein können. Bevorzugt werden Alkylpolyglycoside, EO/PO-Blockcopolymere, Alkyl- oder Arylalkoxylate, Siloxanalkoxylate, Ester der Sulfobernsteinsäure und/oder Alkali- oder Erdalkalimetallalkanoate eingesetzt. Besonders bevorzugt werden EO/PO-Blockcopolymere eingesetzt. Bevorzugt werden allein die EO/PO-Blockcopolymere als Komponente F) eingesetzt.

Zudem können zur Verbesserung der Schaumeigenschaften des resultierenden Polyurethan-Schaums Verbindungen der Komponente G) eingesetzt werden. Hierbei handelt es sich um grundsätzlich alle dem Fachmann an sich bekannten ein- und mehrwertigen Alkohole sowie Mischungen hieraus.

Dies sind ein- oder mehrwertige Alkohole oder Polyole, wie Ethanol, Propanol, Butanol, Decanol, Tridecanol, Hexadecanol, Ethylenglykol, Neopentylglykol, Butandiol, Hexandiol, Decandiol, Trimethylolpropan, Glycerin, Pentaerythrit, monofunktionelle Polyetheralkohole und Polyesteralkohole, Polyetherdiole und Polyesterdiole.

Typischerweise werden die Komponenten A) bis G) in folgenden Mengen eingesetzt:
A) 100 Gewichtsteile isocyanatfunktioneller Präpolymere A)
B) 0 bis 30 Gewichtsteile heterocyclischer Oligomere B)
C) 0,1 bis 200 Gewichtsteile Wasser
D) 0 bis 1 Gewichtteile an Katalysatoren
E) 0,01 bis 5 Gewichtteile an C₈- bis C₁₂-Monocarbonsäuren oder deren Ammonium- oder Alkalisalze oder C₁₂- bis C₄₄-Dicarbonsäuren oder deren Ammonium- oder Alkalisalze
F) 0 bis 10 Gewichtsteile an Tensiden F)
G) 0 bis 20 Gewichtsteile an Alkoholen G)

Bevorzugt werden die Komponenten A) bis G) in folgenden Mengen eingesetzt:
A) 100 Gewichtsteile isocyanatfunktioneller Präpolymere A)
B) 1 bis 30 Gewichtsteile heterocyclischer Oligomere B)
C) 0,1 bis 100 Gewichtsteile Wasser
D) 0,01 bis 1 Gewichtteile an Katalysatoren
E) 0,01 bis 5 Gewichtteile an C₈- bis C₁₂-Monocarbonsäuren oder deren Ammonium- oder Alkalisalze oder C₁₂- bis C₄₄-Dicarbonsäuren oder deren Ammonium- oder Alkalisalze
F) 0 bis 5 Gewichtsteile an Tensiden
G) 0 bis 10 Gewichtsteile an Alkoholen G)

Besonders bevorzugt werden die Komponenten A) bis G) in folgenden Mengen eingesetzt:
A) 100 Gewichtsteile isocyanatfunktioneller Präpolymere A)
B) 5 bis 15 Gewichtsteile heterocyclischer Oligomere B)
C) 1 bis 60 Gewichtsteile Wasser
D) 0,1 bis 0,5 Gewichtteile an Katalysatoren
E) 0,1 bis 1 Gewichtteile an C₈- bis C₁₂-Monocarbonsäuren oder deren Ammonium- oder Alkalisalze oder C₁₂- bis C₄₄-Dicarbonsäuren oder deren Ammonium- oder Alkalisalze

Die Herstellung der erfindungsgemäßen hydrophilen, aliphatischen Polyurethan-Schäume erfolgt durch Mischen der Komponenten A), C), E) und gegebenenfalls B), D), F), G) in beliebiger Reihenfolge, Aufschäumen der Mischung und Aushärtung bevorzugt durch chemische Vernetzung. Bevorzugt werden die Komponenten A) und B) miteinander vorvermischt. Die Carboxylate E) und gegebenenfalls die Tenside F) werden in Form ihrer wässrigen Lösungen dem Reaktionsgemisch zugesetzt.

Das Aufschäumen kann dabei grundsätzlich durch das bei der Reaktion der Isocyanatgruppen mit Wasser gebildete Kohlendioxid erfolgen, die Verwendung von weiteren Treibmitteln ist jedoch ebenfalls möglich. So können prinzipiell auch Treibmittel aus der Klasse der Kohlenwasserstoffe wie C₃-C₆-Alkane, z.B. Butane, n-Pentan, iso-Pentan, cyclo-Pentan, Hexane o.ä. oder halogenierte Kohlenwasserstoffe wie Dichlormethan, Dichlormonofluormethan, Chlordifluorethane, 1,1-Dichlor-2,2,2-Trifluorethan, 2,2-Dichlor-2-fluorethan, insbesondere chlorfreie Fluorkohlenwasserstoffe wie Difluormethan, Trifluormethan, Difluorethan, 1,1,1,2-Tetrafluorethan, Tetrafluorethan (R 134 oder R 134a), 1,1,1,3,3-Pentafluorpropan (R 245 fa), 1,1,1,3,3,3-Hexafluorpropan (R 256), 1,1,1,3,3-Pentafluorbutan (R 365 mfc), Heptafluorpropan oder auch Schwefelhexafluorid verwendet werden. Auch Gemische dieser Treibmittel sind verwendbar.

Die anschließende Aushärtung erfolgt typischerweise bei Raumtemperatur.

Ein weiterer Gegenstand der vorliegenden Erfindung sind die erfindungsgemäßen Zusammensetzungen sowie daraus erhältliche hydrophile, aliphatische Polyurethan-Schäume.

Gegenstand der vorliegenden Erfindung sind überdies die nach dem erfindungsgemäßen Verfahren hergestellten Polyurethan-Schäume, sowie die Verwendung der hydrophilen, aliphatischen Polyurethan-Schäume als Weichschaum, als Bestandteil einer Wundauflage, eines kosmetischen Artikels oder eines Inkontinenzprodukts. Bevorzugt ist jedoch die Verwendung der Polyurethan-Schäume als Bestandteil einer Wundauflage, eines kosmetischen Artikels oder eines Inkontinenzprodukts, besonders bevorzugt ist die Verwendung als Bestandteil einer Wundauflage, ganz besonders bevorzugt als Wundauflage mit direktem Haut- und Wundkontakt der menschlichen oder tierischen Haut.

Die Polyurethan-Schäume weisen eine poröse, zumindest teilweise offenzellige Struktur mit miteinander kommunizierenden Zellen auf. Die Dichte der Polyurethan-Schäume liegt dabei typischerweise bei 0,01 bis 0,5 g/cm³, bevorzugt liegt sie bei 0,02 bis 0,4 g/cm³, besonders bevorzugt bei 0,05 bis 0,3 g/cm³ und ganz besonders bevorzugt bei 0,1 bis 0,2 g/cm³ (Bestimmung nach DIN 53420).

Das Absorptionsvermögen gegenüber physiologischer Salzlösung beträgt bei den PolyurethanSchäumen typischerweise 100 bis 2000 %, bevorzugt 300 bis 2000 %, besonders bevorzugt 800 bis 2000 % und ganz besonders bevorzugt bei 1000 bis 1800 % (Masse der aufgenommenen Flüssigkeit bezogen auf die Masse des trockenen Schaums; Bestimmung nach DIN EN 13726-1, Teil 3.2). Im Vergleich zu anderen hydrophilen Schäumen kann mit den erfindungsgemäßen PolyurethanSchäumen auch ohne die Verwendung von superabsorbierenden Polymeren eine sehr hohe Absorption von physiologischer Salzlösung erreicht werden. Selbstverständlich ist die Einarbeitung von Superabsorbern aber auch bei den erfindungsgemäßen Polyurethan-Schäumen möglich.

Die Polyurethan-Schäume weisen eine gute mechanische Festigkeit und hohe Elastizität auf. Typischerweise sind die Werte für die Zugfestigkeit größer als 40 kPa, für die Bruchdehnung größer als 30 % und für die Rückprallelastizität größer als 60 %. Bevorzugt ist die Zugfestigkeit größer als 50 kPa, die Bruchdehnung größer als 40 % und die Rückprallelastizität größer als 80 % (Bestimmung nach DIN 53504, DIN 53455, DIN EN ISO 3386-1).

Nach der Herstellung können die Polyurethan-Schäume nach an sich bekannten Verfahren zu flächigen Materialien verarbeitet werden, welche dann beispielsweise als Bestandteil einer Wundauflage, eines kosmetischen Artikels oder eines Inkontinenzprodukts eingesetzt werden. In der Regel werden dazu Blockschäume auf die gewünschte Dicke nach gängigen Methoden geschnitten und flächige Materialien mit einer Dicke von typischerweise 10 µ bis 5 cm, bevorzugt 0,1 mm bis 1 cm, besonders bevorzugt 0,1 mm bis 6 mm, ganz besonders bevorzugt 0,2 mm bis 6 mm zu erhalten.

Durch geeignete Gießtechniken können die beschriebenen flächigen Materialien aber auch direkt durch Auftrag und Aufschäumen der erfindungsgemäßen Zusammensetzung auf ein Substrat, z.B. ein gegebenenfalls vorbehandeltes Papier oder Textil, erhalten werden.

Die Polyurethan-Schäume enthalten einen nur geringen mit Wasser extrahierbaren Anteil von maximal 2 Gew.-%, bevorzugt maximal 1 Gew.-%, d.h. sie enthalten nur sehr geringe Mengen an chemisch nicht gebundenen Bestandteilen.

Die Polyurethan-Schäume können überdies mit weiteren Materialien verklebt, laminiert oder beschichtet werden, beispielsweise auf Basis von Hydrogelen, (semi-) permeablen Folien, Schaumfolien, Beschichtungen, Hydrokolloiden oder anderen Schäumen.

Die erfindungsgemäßen Polyurethan-Schäume sind besonders geeignet zur Herstellung von Wundauflagen. Dabei können die Polyurethan-Schäume in direktem oder indirektem Kontakt mit der Wunde sein. Bevorzugt werden die Polyurethan-Schäume jedoch in direktem Kontakt mit der Wunde eingesetzt, um beispielsweise eine optimale Absorption von Wundflüssigkeit zu gewährleisten. Die Polyurethan-Schäume zeigen keine Zelltoxizität (Bestimmung nach ISO 10993-5 und ISO 10993-12).

Die Polyurethan-Schäume, welche als Wundauflage eingesetzt werden, müssen zusätzlich noch in einem weiteren Verfahrensschritt sterilisiert werden. Zur Sterilisation kommen die dem Fachmann an sich bekannten Verfahren zum Einsatz, bei denen eine Sterilisation durch thermische Behandlung, chemische Stoffe wie Ethylenoxid oder Bestrahlung beispielsweise durch Gammabestrahlung erfolgt. Die Bestrahlung kann dabei gegebenenfalls unter Schutzgasatmosphäre erfolgen. Die erfindungsgemäßen Polyurethan-Schäume haben dabei den großen Vorteil, dass sie sich bei Bestrahlung, insbesondere bei Bestrahlung mit Gammastrahlen, nicht verfärben.

Ebenfalls möglich ist die Zugabe, Einarbeitung oder Beschichtung von bzw. mit antimikrobiellen oder biologischen Wirkstoffen, welche sich beispielsweise in Bezug auf die Wundheilung und die Vermeidung von Keimbelastungen positiv auswirken.

### Beispiele

Sofern nicht abweichend gekennzeichnet, beziehen sich alle Prozentangaben auf das Gewicht. Die Bestimmung der Festkörpergehalte erfolgte nach DIN-EN ISO 3251. Die Bestimmung der Viskositäten erfolgte bei 23 °C und wurde nach DIN 53019 durchgeführt. Die NCO-Gehalte wurden volumetrisch gemäß DIN-EN ISO 11909 bestimmt.

### Verwendete Substanzen und Abkürzungen:

- Carboxylat 1:: 10 % Natriumoleat in Wasser
- Carboxylat 2:: 10 % Natrium-2-Ethylhexanoat in Wasser
- Dispergiermittel EM:: Polyetherpolyol der OH-Zahl 70 mg KOH/g (Rhein Chemie Rheinau GmbH, Mannheim, DE)
- Zusatzmittel VP.PU 3240:: Polyglykolester der OH-Zahl 100 mg KOH/g (Rhein Chemie Rheinau GmbH, Mannheim, DE)
- Tegostab^{®} B 2370:: Polysiloxan-polyoxyalkylen Blockcopolymer (Degussa-Goldschmidt AG, Essen, DE)
- Desmodur^{®} N 3400:: Aliphatisches Polyisocyanat (HDI-Uretdion), NCO-Gehalt 21,8 %
- Desmodur^{®} N 3600:: Aliphatisches Polyisocyanat (HDI-Isocyanurat), NCO-Gehalt 24 %
- Pluronic^{®} PE 3500:: EO/PO-Blockcopolymer (BASF, Ludwigshafen, DE)
- Pluronic^{®} PE 6800:: EO/PO-Blockcopolymer (BASF, Ludwigshafen, DE)
- Desmophen^{®} 41 WB01:: Polyetherpolyol der OH-Zahl 37 mg KOH/g (Bayer MaterialScience AG, Leverkusen, DE)
- Polyether PW 56:: Polyetherpolyol der OH-Zahl 56 mg KOH/g (Bayer MaterialScience AG, Leverkusen, DE)
- Polyether PEG 400:: Polyetherpolyol der OH-Zahl 280 mg KOH/g (BASF AG, Ludwigshafen, DE)
- Polyether LB 25:: monofunktioneller Polyether auf Ethylenoxid-/Propylenoxidbasis, zahlenmittleres Molekulargewicht 2250 g/mol, OH-Zahl 25 mg KOH/g (Bayer MaterialScience AG, Leverkusen, DE)

### Beispiel 1: Herstellung des Polyurethan-Präpolymers_1

Zu einem Gemisch aus 1000g HDI und 1g Benzoylchlorid wurde bei 80°C innerhalb von 3h 1000g eines Polyalkylenoxids mit einer Molmasse von 4680 g/mol gestartet auf Glycerin, einem Ethylenoxidgewichtsanteil von 72 % und Propylenoxidgewichtsanteil von 28 %, das zuvor bei 100°C während 6h bei einem Druck von 0,1 mbar getrocknet wurde, zugetropft und für 12h nachgerührt. Das überschüssige HDI wurde durch Dünnschichtdestillation bei 130°C und 0,1 mbar entfernt, wobei die nicht flüchtigen Bestandteile mit 1g Chlorpropionsäure stabilisiert wurden. Man erhielt ein Präpolymer mit einem NCO-Gehalt von 2,77 % und einer Viskosität von 3500 mPas.

### Beispiel 2: Herstellung des Polyurethan-Präpolymers 2

Zu einem Gemisch aus 200g HDI, 1 g Benzoylchlorid und 1g Methyltosylat wurde bei 80°C innerhalb von 2h 400g eines Polyalkylenoxids mit einer Molmasse von 5800 g/mol gestartet auf Glycerin, einem Ethylenoxidgehalt von 80 % und einem Propylenoxidgehalt von 20 %, das vorher bei 100°C während 6h bei einem Druck von 0,1 mbar getrocknet wurde, zugetropft und für 12h nachgerührt. Das überschüssige HDI wurde durch Dünnschichtdestillation bei 130°C und 0,1 mbar entfernt. Man erhielt ein Präpolymer mit einem NCO-Gehalt von 2,31 % und einer Viskosität von 6070 mPas.

### Beispiel 3: Herstellung des Polyurethan-Präpolymers 3

Zu einem Gemisch aus 1440g HDI und 4g Benzoylchlorid wurde bei 80°C innerhalb von 2h 2880g eines Polyalkylenoxids mit einer Molmasse von 4680 g/mol gestartet auf Glycerin, einem Ethylenoxidgewichtsanteil von 72 % und Propylenoxidgewichtsanteil von 28 %, das zuvor bei 100°C während 6h bei einem Druck von 0,1 mbar getrocknet wurde, zugetropft und für 1h nachgerührt. Das überschüssige HDI wurde durch Dünnschichtdestillation bei 130°C und 0,1 mbar entfernt. Man erhielt ein Präpolymer mit einem NCO-Gehalt von 2,11 % und einer Viskosität von 3780 mPas.

### Beispiel 4: Herstellung des Polyurethan-Präpolymers 4

Zu einem Gemisch aus 200g IPDI, 1g Benzoylchlorid und 1g Methyltosylat wurde bei 80°C innerhalb von 2h 400g eines Polyalkylenoxids mit einer Molmasse von 5800 g/mol gestartet auf Glycerin, einem Ethylenoxidgehalt von 80 % und einem Propylenoxidgehalt von 20 %, das vorher bei 100°C während 6h bei einem Druck von 0,1 mbar getrocknet wurde, zugetropft und für 12 h nachgerührt. Das überschüssige IPDI wurde durch Dünnschichtdestillation bei 130°C und 0,1 mbar entfernt. Man erhielt ein Präpolymer mit einem NCO-Gehalt von 2,36 % und einer Viskosität von 8800 mPas.

### Beispiele 5-13: Herstellung von Schaumstoffen aus den Polyurethan-Präpolymeren 1-3

Die beiden Isocyanat-Komponenten wurden 15 Sekunden mit einer Rührerdrehzahl von 1200 Upm homogenisiert, dann die weiteren Komponenten eingewogen, weitere 10 Sekunden verrührt und in einen Becher vom Volumen 500 ml eingebracht.

| **Komponente [g]** | **Beispiel** | | | | | | | | |
|---|---|---|---|---|---|---|---|---|---|
| | **5** | **6** | **7** | **8** | **9** | **10** | **11** | **12** | **13** |
| Präpolymer | 36,0¹⁾ | 36,0²⁾ | 36,0²⁾ | 36,0²⁾ | 36,0²⁾ | 20,0²⁾ | 20,0²⁾ | 20,0²⁾ | 20,0²⁾ |
| Oligomer | 4,0⁴⁾ | 4,0⁴⁾ | 4,0⁴⁾ | 4,0⁴⁾ | 4,0⁴⁾ | 2,2⁵⁾ | 2,2⁴⁾ | | 2,2⁴⁾ |
| Additiv | 0,6⁶⁾ | 0,6⁶⁾ | 1,2⁷⁾ | | 0,6⁸⁾ | | 0,4⁹⁾ | 0,4⁹⁾ | 0,4⁹⁾ |
| DBU | 0,05 | 0,05 | 0,05 | 0,05 | 0,05 | 0,03¹⁰⁾ | 0,03 | 0,03¹⁰⁾ | 0,03¹⁰⁾ |
| Carboxylat | 2,0¹¹⁾ | 2,0¹²⁾ | 2,0¹¹⁾ | 2,0¹¹⁾ | 2,0¹¹⁾ | 1,1¹¹⁾ | 1,1¹¹⁾ | 1,1¹¹⁾ | 1,1¹¹⁾ |
| | | | | | | | | | |
| Startzeit [s] | 7 | 35 | 30 | 15 | 26 | 28 | 20 | 30 | 20 |
| Rohdichte [g/cm³] | 0,12 | 0,12 | 0,12 | 0,12 | 0,13 | 0,14 | 0,17 | 0,27 | 0,13 |

| | | | | | | | | | |
|---|---|---|---|---|---|---|---|---|---|
| ¹⁾ Präpolymer 2; ²⁾ Präpolymer 1; ³⁾ Präpolymer 3; ⁴⁾ Desmodur N 3400; ⁵⁾ Desmodur N 3600; ⁶⁾ Dispergiermittel EM; ⁷⁾ Zusatzmittel VP.PU 3240/Tegostab^{®} B 2370 (je 50 %); ⁸⁾ Pluronic^{®} PE 3500; ⁹⁾ Pluronic^{®} PE 6800; ¹⁰⁾ gelöst in 0,5 g Desmophen^{®} 41WB01; ¹¹⁾ Carboxylat 1; ¹²⁾ Carboxylat 2 | | | | | | | | | |

In den Beispielen 5 bis 13 wurden Schaumstoffe von regelmäßig feiner Zellstruktur erhalten, die dimensionsstabil und elastisch sind. Sie zeigen nach Belastung ein sehr starkes Rückstellvermögen und einen geringen Druckverformungsrest mit einer relativ niedrigen Stauchhärte von 1-5 kPa bei 40 % Stauchung bei höheren Rohdichten. Dies ist wichtig für eine gute Aufnahmekapazität an Wundsekret und der Anpassung an Konturen. Exemplarisch wurde gemäß Richtlinie ISO 10993.5 gezeigt, dass der aus Beispiel 13 resultierende Schaum als nicht zytotoxisch einzustufen ist.

Wie Beispiel 12 zeigt, resultieren bei Verzicht auf die heterocyclischen Oligomere B) kompaktere Polyurethanschäume.

### Vergleichsbeispiel 1: Carboxylatfreie Umsetzung der Präpolymere

Unter vergleichbaren Bedingungen wie in den Beispielen 5-13 wurden zunächst 20,0 g Präpolymer 1 und 2,2 g Desmodur N 3400 homogenisiert und anschließend mit einer Lösung von 0,03 g DBU in 1,0 g Wasser versetzt. Nach einer Startzeit von 20 Sekunden begann die Schaumexpansion, jedoch erleidete der resultierende Schaum starken Schrumpf. Auch die Zugabe von an sich bekannten Schaumhilfsmitteln wie beispielsweise Pluronic^{®} PE 6800 zur wässrigen Lösung des Katalysators verhindert diesen Schrumpf nicht.

Wie dieses Vergleichsbeispiel verdeutlicht, stellen die erfindungsgemäßen Carboxylate eine maßgebliche Komponente in der beschriebenen Schaumherstellung dar: Ohne diese Salze kommt es nach dem Aufschäumen der Polyurethanschäume - auch trotz Zugabe allgemein hin bekannter Schaumadditive - zu starkem Schrumpf, was produktionstechnisch nicht bevorzugt ist. Ebenso wird bei der Verwendung entsprechender Alkali-Sulfate oder -Phosphate starker Schrumpf bei den resultierenden Polyurethanschäumen beobachtet.

### Beispiele 14-17: Herstellung von Schaumstoffen aus Polyurethan-Präpolymer 1

20,0 g Präpolymer 1 und 2,2 g Desmodur^{®} N 3400 wurden 15 Sekunden mit einer Rührerdrehzahl von 1200 Upm homogenisiert, dann 0,03 g DBU gelöst in 0,5 g Desmophen^{®} 41WB01, 1,1 g Carboxylat 1 und 0,2 g der Alkoholkomponente eingewogen, weitere 10 Sekunden verrührt und in einen Becher vom Volumen 250 ml eingebracht.

| | **Beispiel** | | | |
|---|---|---|---|---|
| | **14** | **15** | **16** | **17** |
| Alkohol | 1,4-Butandiol | Polyether PEG 400 | Polyether PW 56 | Polyether LB 25 |
| Startzeit [s] | 20 | 21 | 20 | 30 |
| Rohdichte [g/cm³] | 0,13 | 0,13 | 0,12 | 0,16 |

Wie die Beispiele 14 bis 17 verdeutlichen, wurden auch nach Abmischung der erfindungsgemäßen wässrigen Carboxylate mit Diolen Schaumstoffe von regelmäßig feiner Zellstruktur erhalten, die dimensionsstabil und elastisch sind.

### Beispiele 18-24: Herstellung von Schaumstoffen aus Polyurethan-Präpolymer 1

20,0 g Präpolymer 1 und 2,2 g Desmodur^{®} N 3400 wurden 15 Sekunden mit einer Rührerdrehzahl von 1200 Upm homogenisiert, dann 0,03 g Katalysator und 1,1 g Carboxylat 1 eingewogen, weitere 10 Sekunden verrührt und in einen Becher vom Volumen 250 ml eingebracht.

| | **Beispiel** | | | | | | |
|---|---|---|---|---|---|---|---|
| | **18** | **19** | **20** | **21** | **22** | **23** | **24¹⁾** |
| Katalysator | DBU | DABCO | SO | ZO | TMG | - | - |
| Startzeit [s] | 20 | 40 | 30 | 60 | 40 | 50 | 120 |
| Rohdichte [g/cm³] | 0,13 | 0,11 | 0,13 | 0,13 | 0,16 | 0,13 | 0,11 |
| Zellstruktur | fein | fein | fein | sehr grob | fein | mittel | fein |

| | | | | | | | |
|---|---|---|---|---|---|---|---|
| ¹⁾ Carboxylat 1 ersetzt durch 1,0 g einer 2%igen wässrigen Natriumoleat-Lösung | | | | | | | |

Wie die Beispiele 18 bis 22 verdeutlichen, konnte mit der Wahl des Katalysators neben der Reaktionsgeschwindigkeit auch Einfluss auf die Zellstruktur genommen werden. Zudem verdeutlichen die Beispiele 23 und 24, dass Carboxylat 1 auch katalytische Eigenschaften besitzt.

### Beispiel 25: Herstellung eines Schaumstoffs aus dem Polyurethan-Präpolymer 4

Zur Ermittlung von Stauchhärte nach DIN 53577 und der Rohdichte nach DIN 53420 wurden in einem 1000 ml Polypropylen-Gefäß 108 g Präpolymer 4 und 12 g Desmodur N 3400 für 15 Sekunden mit einer Rührerdrehzahl von 1200 Upm homogenisiert. Anschließend wurden 1,8 g Dispergiermittel EM, 0,15 g DBU und 3 g Carboxylat 1 zugesetzt und das Gemenge für weitere 10 Sekunden verrührt. Der resultierende, ausgehärtete Schaum zeigte keine große Haftung an der Gefäßwandung. Er besaß bei 40 % Stauchung eine Stauchhärte von 3,1 kPa sowie eine Rohdichte von 0,08 g/cm³.

### Beispiel 26: Bestimmung der Extraktmenge von Polyurethanschaum 5

10 g des Schaums aus Beispiel 5 wurden für 48 Stunden in 300 ml vollentsalztes Wasser bei 36°C eingelegt und durch Titration des chemischen Sauerstoffbedarfs gemäß DIN EN 1484 die Extraktmenge bestimmt. Sie betrug 0,6 Gew.-%.

### Beispiel 27: Bestimmung der Extraktmenge von Polyurethanschaum 8

4,7 g des Schaums aus Beispiel 8 wurden für 7 Tage in 220 ml vollentsalztes Wasser bei 37°C eingelegt und durch Titration des chemischen Sauerstoffbedarfs gemäß DIN EN 1484 die Extraktmenge bestimmt. Sie betrug 0,2 Gew.-%.

Beispiel 24 der US 5065752, das einzige Beispiel, in dem zumindest eine Teilmenge des Isocyanats aliphatisch war, beschreibt einen Schaum mit einem Extraktgehalt von 30 Gew.-%.

### Beispiel 28 bis 30: Formulierung mit überschussigem Wasser

Beispiel 29 und 30 zeigen im Vergleich zu Beispiel 28 (kein extra Wasser) dass durch Zugabe von überschüssigen Mengen Wasser in der Formulierung, die Verarbeitungszeit (Giesszeit) verlängert wird und so extrem feinporige homogene Schäume erhalten werden.Durch die Verdünnung mit Wasser werden dünnere, aber sehr homogene Schäume erhalten, die besonders geeignet sind für Wundauflagen.

| **Komponente [g]** | | | | | |
|---|---|---|---|---|---|
| | **28** | | **29** | | **30** |
| Präpolymer | 20,0¹⁾ | | 20,0¹⁾ | | 20,0¹⁾ |
| Oligomer | 2,2²⁾ | | 2,2²⁾ | | 2,2 2²⁾ |
| DBU | 0,027 | | 0,027 | | 0,027 |
| Carboxylat | 1,1³⁾ | | 1,1³⁾ | | 1,1³⁾ |
| Wasser | 0 | | 4 | | 10 |
| | | | | | |
| Startzeit [s] | 25 | | 40 | | 60 |
| Giesszeit (s) | 35 | | 50 | | 70 |
| Rohdichte [g/cm³] | 0,17 | | 0.26 | | 0,40 |
| Schaumdicke nach Giessen (mm) | 15 | | 8 | | 4 |
| Porenqualität | Fein aber nicht homogen, stellenweise grobe Poren | | Sehr fein und Homogen | | Sehr fein und Homogen |

| | | | | | |
|---|---|---|---|---|---|
| ¹⁾ Präpolymer 2 ²⁾Desmodur N 3400; ³⁾Carboxylat 1, 10%-ige Lösung in Wasser | | | | | |

## Patentansprüche

1. Verfahren zur Herstellung hydrophiler, aliphatischer Polyurethan-Schäume, bei dem Zusammensetzungen umfassend
A) isocyanatfunktionelle Präpolymere mit einem Gewichtsanteil an niedermolekularen, aliphatischen Diisocyanaten mit einer Molmasse von 140 bis 278 g/mol von unter 1,0 Gew.-% bezogen auf das Präpolymer, erhältlich durch Umsetzung von
A1) niedermolekularen, aliphatischen Diisocyanaten einer Molmasse von 140 bis 278 g/mol mit
A2) di- bis hexafunktionellen Polyalkylenoxiden einer OH-Zahl von 22,5 bis 112 mg KOH/g, und einem Ethylenoxidanteil von 50 bis 100 mol% bezogen auf die Gesamtmenge der enthaltenen Oxyalkylengruppen,
B) gegebenenfalls Verbindungen, die heterocyclische 4-Ring- oder 6-Ring-Oligomere von niedermolekularen, aliphatischen Diisocyanaten mit einer Molmasse von 140 bis 278 g/mol sind,
C) Wasser,
D) gegebenenfalls Katalysatoren,
E) C₈- bis C₂₂-Monocarbonsäuren oder deren Ammonium- oder Alkalisalze oder C₁₂- bis C₄₄-Dicarbonsäuren oder deren Ammonium- oder Alkalisalze,
F) gegebenenfalls Tenside und
G) gegebenenfalls ein- oder mehrwertige Alkohole
bereitgestellt, aufgeschäumt und ausgehärtet werden.

2. Verfahren gemäß Anspruch 1, **dadurch gekennzeichnet, dass** der NCO-Gehalt der isocyanatfunktionellen Präpolymere A) 1,5 bis 3,0 Gew.-% beträgt.

3. Verfahren gemäß Anspruch 1 oder 2, **dadurch gekennzeichnet, dass** in A1) ausschließlich HDI, IPDI oder deren Mischungen untereinander eingesetzt werden.

4. Verfahren gemäß einem der Ansprüche 1 bis 3, **dadurch gekennzeichnet, dass** in A2) als Polyalkylenoxide Copolymere aus Ethylenoxid und Propylenoxid mit einem Ethylenoxidgehalt, bezogen auf die Gesamtmenge der enthaltenen Oxyalkylengruppen, von 60 bis 85 mol%, gestartet auf Polyolen oder Aminen eingesetzt werden.

5. Verfahren gemäß einem der Ansprüche 1 bis 4, **dadurch gekennzeichnet, dass** die Polyalkylenoxide der Komponente A2) zahlenmittlere Molekulargewichte von 3000 bis 8500 g/mol aufweisen.

6. Verfahren gemäß einem der Ansprüche 1 bis 5, **dadurch gekennzeichnet, dass** die Polyalkylenoxide der Komponente A2) OH-Funktionalitäten von 3 bis 4 aufweisen.

7. Verfahren gemäß einem der Ansprüche 1 bis 6, **dadurch gekennzeichnet, dass** in Komponente B) heterocyclische 4-Ring-Oligomere eingesetzt werden.

8. Verfahren gemäß einem der Ansprüche 1 bis 7, **dadurch gekennzeichnet, dass** als Katalysatoren der Komponente D) Metallsalze, Amine, Amidine und Guanidine eingesetzt werden.

9. Verfahren gemäß einem der Ansprüche 1 bis 7, **dadurch gekennzeichnet, dass** die Komponenten A) bis E) in den folgenden Mengen eingesetzt werden:
100 Gewichtsteile isocyanatfunktioneller Präpolymere A)
5 bis 15 Gewichtsteile heterocyclischer Oligomere B)
1 bis 200 Gewichtsteile Wasser C)
0,1 bis 0,5 Gewichtteile an Katalysatoren D)
0,1 bis 1 Gewichtteile an C₈- bis C₁₂-Monocarbonsäuren oder deren Ammonium- oder Alkalisalze oder C₁₂- bis C₄₄-Dicarbonsäuren oder deren Ammonium- oder Alkalisalze als Komponente E)

10. Zusammensetzungen umfassend
A) isocyanatfunktionelle Präpolymere mit einem Gewichtsanteil an niedermolekularen, aliphatischen Diisocyanaten mit einer Molmasse von 140 bis 278 g/mol von unter 1,0 Gew.-% bezogen auf das Präpolymer, erhältlich durch Umsetzung von
A1) niedermolekularen, aliphatischen Diisocyanaten einer Molmasse von 140 bis 278 g/mol mit
A2) di- bis hexafunktionellen, bevorzugt tri- bis hexafunktionellen Polyalkylenoxiden einer OH-Zahl von 22,5 bis 112, bevorzugt von 31,5 bis 56, und einem Ethylenoxidanteil von 50 bis 100 mol%, bevorzugt 60 bis 85 mol%, bezogen auf die Gesamtmenge der enthaltenen Oxyalkylengruppen,
B) gegebenenfalls Verbindungen, die heterocyclische, 4-Ring- oder 6-Ring-Oligomere von niedermolekularen, aliphatischen Diisocyanaten mit einer Molmasse von 140 bis 278 g/mol sind,
C) Wasser,
D) gegebenenfalls Katalysatoren,
E) C₈- bis C₂₂-Monocarbonsäuren oder deren Ammonium- oder Alkalisalze oder C₁₂- bis C₄₄-Dicarbonsäuren oder deren Ammonium- oder Alkalisalze,
F) gegebenenfalls Tenside und
G) gegebenenfalls ein- oder mehrwertige Alkohole

11. Polyurethanschäume erhältlich nach einem Verfahren gemäß einem der Ansprüche 1 bis 9 oder aus Zusammensetzungen gemäß Anspruch 10.

12. Wundauflagen, kosmetische Artikel oder Inkontinenzprodukte erhältlich unter Verwendung von Polyurethanschäumen gemäß Anspruch 11.

## Claims

1. Process for preparing hydrophilic aliphatic polyurethane foams wherein compositions comprising
A) isocyanate-functional prepolymers having a weight fraction of low molecular weight aliphatic diisocyanates having a molar mass of 140 to 278 g/mol of below 1.0% by weight based on the prepolymer, obtainable by reaction of
A1) low molecular weight aliphatic diisocyanates having a molar mass of 140 to 278 g/mol with
A2) di- to hexafunctional polyalkylene oxides having an OH number of 22.5 to 112 mg KOH/g and an ethylene oxide content of 50 to 100 mol% based on the total amount of oxyalkylene groups present,
B) optionally compounds which are heterocyclic 4-ring or 6-ring oligomers of low molecular weight aliphatic diisocyanates having a molar mass of 140 to 278 g/mol,
C) water,
D) optionally catalysts,
E) C₈-C₂₂ monocarboxylic acids or their ammonium or alkali metal salts or C₁₂-C₄₄ dicarboxylic acids or their ammonium or alkali metal salts,
F) optionally surfactants, and
G) optionally mono- or polyhydric alcohols,
are provided, foamed and cured.

2. Process according to Claim 1, **characterized in that** the NCO content of the isocyanate-functional prepolymers A) is 1.5% to 3.0% by weight.

3. Process according to Claim 1 or 2, **characterized in that** A1) exclusively utilizes HDI, IPDI or mixtures therebetween.

4. Process according to any one of Claims 1 to 3, **characterized in that** polyalkylene oxides in A2) comprise copolymers of ethylene oxide and propylene oxide having an ethylene oxide content, based on the total amount of oxyalkylene groups present, of 60 to 85 mol% and started on polyols or amines.

5. Process according to any one of Claims 1 to 4, **characterized in that** the polyalkylene oxides of component A2) have number average molecular weights of 3000 to 8500 g/mol.

6. Process according to any one of Claims 1 to 5, **characterized in that** the polyalkylene oxides of component A2) have OH functionalities of 3 to 4.

7. Process according to any one of Claims 1 to 6, **characterized in that** heterocyclic 4-ring oligomers are used in component B).

8. Process according to any one of Claims 1 to 7, **characterized in that** metal salts, amines, amidines and guanidines are used as catalysts of component D).

9. Process according to any one of Claims 1 to 7, **characterized in that** components A) to E) are used in the following amounts:
100 parts by weight of isocyanate-functional prepolymers A)
5 to 15 parts by weight of heterocyclic oligomers B)
1 to 200 parts by weight of water C)
0.1 to 0.5 parts by weight of catalysts D)
0.1 to 1 part by weight of C₈-C₁₂ monocarboxylic acids or their ammonium or alkali metal salts or C₁₂-C₄₄ dicarboxylic acids or their ammonium or alkali metal salts as component E).

10. Compositions comprising
A) isocyanate-functional prepolymers having a weight fraction of low molecular weight aliphatic diisocyanates having a molar mass of 140 to 278 g/mol of below 1.0% by weight based on the prepolymer, obtainable by reaction of
A1) low molecular weight aliphatic diisocyanates having a molar mass of 140 to 278 g/mol with
A2) di- to hexafunctional, preferably tri- to hexafunctional polyalkylene oxides having an OH number of 22.5 to 112 , preferably from 31.5 to 56, and an ethylene oxide content of 50 to 100 mol%, preferably from 60 to 85 mol %, based on the total amount of oxyalkylene groups present,
B) optionally compounds which are heterocyclic 4-ring or 6-ring oligomers of low molecular weight aliphatic diisocyanates having a molar mass of 140 to 278 g/mol,
C) water,
D) optionally catalysts,
E) C₈-C₂₂ monocarboxylic acids or their ammonium or alkali metal salts or C₁₂-C₄₄ dicarboxylic acids or their ammonium or alkali metal salts,
F) optionally surfactants, and
G) optionally mono- or polyhydric alcohols.

11. Polyurethane foams obtainable by following a process according to any one of Claims 1 to 9 or from compositions according to Claim 10.

12. Wound dressings, cosmetic articles or incontinence products obtainable using polyurethane foams according to Claim 11.

## Revendications

1. Procédé pour la production de mousses de polyuréthane aliphatiques, hydrophiles, dans lequel on prépare, transforme en mousse et fait durcir des compositions comprenant
A) des prépolymères à fonction isocyanate ayant une teneur pondérale en diisocyanates aliphatiques de faible masse moléculaire, ayant une masse moléculaire de 140 à 278 g/mole, de moins de 1,0 % en poids par rapport au prépolymère, pouvant être obtenus par mise en réaction
A1) de diisocyanates aliphatiques de faible masse moléculaire, ayant une masse moléculaire de 140 à 278 g/mole, avec
A2) des polyoxyalkylènes di- à hexafonctionnels ayant un indice de groupes OH de 22,5 à 112 mg de KOH/g, et une teneur en oxyde d'éthylène de 50 à 100 % en moles par rapport à la quantité totale des groupes oxyalkylène contenus,
B) éventuellement des composés qui sont des oligomères hétérocycliques à cycle tétragonal ou hexagonal de diisocyanates aliphatiques de faible masse moléculaire ayant une masse moléculaire de 140 à 278 g/mole,
C) de l'eau,
D) éventuellement des catalyseurs,
E) des acides monocarboxyliques en C₈-C₂₂ ou leurs sels alcalins ou d'ammonium ou des acides dicarboxyliques en C₁₂-C₄₄ ou leurs sels alcalins ou d'ammonium,
F) éventuellement des tensioactifs et
G) éventuellement des alcools mono- ou polyhydriques.

2. Procédé selon la revendication 1, **caractérisé en ce que** la teneur en NCO des prépolymères A) à fonction isocyanate vaut de 1,5 à 3,0 % en poids.

3. Procédé selon la revendication 1 ou 2, **caractérisé en ce que** dans A1) on utilise exclusivement du HDI, de l'IPDI ou des mélanges de ceux-ci entre eux.

4. Procédé selon l'une quelconque des revendications 1 à 3, **caractérisé en ce que** dans A2) on utilise comme polyoxyalkylènes des copolymères d'oxyde d'éthylène et oxyde de propylène ayant une teneur en oxyde d'éthylène, par rapport à la quantité totale des groupes oxyalkylène contenus, de 60 à 85 % en moles, amorcés sur des polyols ou des amines.

5. Procédé selon l'une quelconque des revendications 1 à 4, **caractérisé en ce que** les polyoxyalkylènes du composant A2) présentent des masses moléculaires moyennes en nombre de 3 000 à 8 500 g/mole.

6. Procédé selon l'une quelconque des revendications 1 à 5, **caractérisé en ce que** les polyoxyalkylènes du composant A2) présentent des fonctionnalités OH de 3 à 4.

7. Procédé selon l'une quelconque des revendications 1 à 6, **caractérisé en ce que** dans le composant B) on utilise des oligomères hétérocycliques à cycle tétragonal.

8. Procédé selon l'une quelconque des revendications 1 à 7, **caractérisé en ce que** comme catalyseurs du composant D) on utilise des sels métalliques, des amines, des amidines et des guanidines.

9. Procédé selon l'une quelconque des revendications 1 à 7, **caractérisé en ce qu'**on utilise les composants A) à E) en les quantités suivantes :
100 parties en poids de prépolymères A) à fonction isocyanate
5 à 15 parties en poids d'oligomères hétérocycliques B)
1 à 200 parties en poids d'eau C)
0,1 à 0,5 partie en poids de catalyseurs D)
0,1 à 1 partie en poids d'acides monocarboxyliques en C₈-C₁₂ ou de leurs sels alcalins ou d'ammonium ou d'acides dicarboxyliques en C₁₂-C₄₄ ou de leurs sels alcalins ou d'ammonium, en tant que composant E).

10. Compositions comprenant
A) des prépolymères à fonction isocyanate ayant une teneur pondérale en diisocyanates aliphatiques de faible masse moléculaire, ayant une masse moléculaire de 140 à 278 g/mole, de moins de 1,0 % en poids par rapport au prépolymère, pouvant être obtenus par mise en réaction
A1) de diisocyanates aliphatiques de faible masse moléculaire, ayant une masse moléculaire de 140 à 278 g/mole, avec
A2) des polyoxyalkylènes di- à hexafonctionnels, de préférence tri- à hexafonctionnels, ayant un indice de groupes OH de 22,5 à 112, de préférence de 31,5 à 56, et une teneur en oxyde d'éthylène de 50 à 100 % en moles, de préférence de 60 à 85 % en moles, par rapport à la quantité totale des groupes oxyalkylène contenus
B) éventuellement des composés qui sont des oligomères hétérocycliques à cycle tétragonal ou hexagonal de diisocyanates aliphatiques de faible masse moléculaire ayant une masse moléculaire de 140 à 278 g/mole,
C) de l'eau,
D) éventuellement des catalyseurs,
E) des acides monocarboxyliques en C₈-C₂₂ ou leurs sels alcalins ou d'ammonium ou des acides dicarboxyliques en C₁₂-C₄₄ ou leurs sels alcalins ou d'ammonium,
F) éventuellement des tensioactifs et
G) éventuellement des alcools mono- ou polyhydriques.

11. Mousses de polyuréthane pouvant être obtenues conformément à un procédé selon l'une quelconque des revendications 1 à 9 ou à partir de compositions selon la revendication 10.

12. Pansements, articles cosmétiques ou produits pour incontinence, pouvant être obtenus avec utilisation de mousses de polyuréthane selon la revendication 11.
